# EUROPEAN PATENT APPLICATION

(11) **EP 3 521 410 A1**
(43) Date of publication of application: **07.08.2019**
(21) Application number: 17855906.8
(22) Date of filing: 20.09.2017
(51) Int. Cl.: C12M 1/00, C12M 1/34, G01N 21/17, G02B 21/06

(54) **CULTURING OBSERVING SYSTEM**

(30) Priority: 30.09.2016 JP 2016192716
(71) Applicant: Olympus Corporation, Hachioji-shi Tokyo 192-8507 (JP)
(72) Inventor: TAKIMOTO, Shinichi, Hachioji-shi Tokyo 192-8507 (JP); MINE, Taiji, Hachioji-shi Tokyo 192-8507 (JP); NAKATOMI, Takayuki, Hachioji-shi Tokyo 192-8507 (JP)
(74) Representative: Winter, Brandl, Fürniss, Hübner, Röss, Kaiser, Polte - Partnerschaft mbB
(86) International application number: PCT/JP2017/033979
(87) International publication number: WO 2018/061951

(57) **Abstract**

The burden of having to perform a confirmation task at the time of cell culturing is reduced. Provided is a culture observation system including: an observation device that is disposed in an incubator and that is used to observe a sample in a culture container; a station server that is disposed outside the incubator and that transmits and receives information to and from the observation device; and a terminal for transmitting and receiving information to and from the station server, wherein the observation device includes: a light source unit for emitting illumination light from below the sample to thereabove, said sample being accommodated in the container formed of an optically transparent material; and an image acquisition unit that acquires, below the sample, an image of transmitted light that has passed through the sample as a result of illumination light emitted from the light source unit being reflected at an inner surface of a top plate of the container disposed above the sample.

## Description

### {Technical Field}

The present invention relates to a culture observation system.

### {Background Art}

For conventional cell culturing, the task of taking a culture container out of an incubator, peeling cells off the culture container, and seeding and culturing cells in a new culture container is repeated at predetermined intervals each time cells become confluent (refer to, for example, Patent Literature 1).

### {Citation List}

### {Patent Literature}

{PTL 1} Japanese Unexamined Patent Application, Publication No. 6-217989

### {Summary of Invention}

### {Technical Problem}

However, there is a problem in that, during the time period between these tasks, an observer is required to carry out a very burdensome confirmation task of taking out the culture container from the incubator and confirming culture conditions with, for example, a microscope at predetermined time intervals (e.g., once or twice a day).

The present invention has been conceived in light of the above-described circumstances, and an object thereof is to provide a culture observation device that can reduce the burden of the confirmation task at the time of cell culturing.

### {Solution to Problem}

In order to achieve the above-described object, the present invention provides the following solutions.

One aspect of the present invention is a culture observation system including: an observation device that is disposed in an incubator and that is used to observe a sample in a culture container; a station server that is disposed outside the incubator and that transmits and receives information to and from the observation device; and a terminal for transmitting and receiving information to and from the station server, wherein the observation device includes: a light source unit for emitting illumination light from below the sample to thereabove, said sample being accommodated in the container formed of an optically transparent material; and an image acquisition optical system for acquiring, below the sample, an image of transmitted light that has passed through the sample as a result of the illumination light emitted from the light source unit being reflected at an inner surface of a top plate of the container disposed above the sample.

According to this aspect, the illumination light emitted from the light source unit passes through the sample from below the sample to thereabove, is reflected above the sample, and passes through the sample from above the sample to therebelow. An image of transmitted light that has passed through the sample is acquired by the image acquisition optical system disposed below the sample. Because both the light source unit and the image acquisition optical system are disposed below the sample, observation is possible without having to increase the size of the device by acquiring an image of transmitted light without labeling a subject, such as cells. Also, because a transmission unit transmits the acquired image to the outside of the incubator, it is possible to confirm the culture state of the cells in the culture container without opening the door of the incubator and taking out the culture container. By doing so, it is possible to reduce the burden at the time of cell culturing.

In the above-described aspect, the station server and the terminal may transmit and receive information via a cloud server.

By doing so, a user can remotely confirm the culture state of the cells in the culture container and can remotely operate the observation device in the incubator.

In the above-described aspect, transmission and reception of information from the station server to the cloud server may be performed according to each instruction from the terminal. In the above-described aspect, transmission and reception of information from the station server to the cloud server may be automatically performed at predetermined intervals and/or for predetermined image data.

In the above-described aspect, the image acquisition optical system may include an objective lens for collecting the transmitted light that has passed through the sample, and the light source unit may emit illumination light from radially outward of the objective lens to above the sample.

By doing so, the illumination light emitted towards an area above the sample from the light source unit that is disposed radially outwardly of the objective lens disposed below the sample is reflected above the sample and is incident obliquely downward on the sample relative to the optical axis of the objective lens, whereby an image of the transmitted light that has passed through the sample is acquired by the image acquisition optical system. Light and dark areas can be formed on an image of the sample by appropriately setting the angle of incidence on the sample, thereby making it possible to acquire an easy-to-see image even for a transparent subject, such as cells.

In the above-described aspect, the light source unit may be capable of independently emitting illumination light from different positions in the radial direction of the objective lens.

By doing so, the angle of incidence, on the sample, of reflection light reflected by the same reflecting surface disposed above the sample can be changed by making radial positions of illumination light emitted from the light source unit different. More specifically, reflection light of light emitted from a nearby position in the radial direction of the objective lens is incident on the sample at a small angle relative to the optical axis, whereas reflection light of illumination light emitted from a distant position in the radial direction of the objective lens is incident on the sample at a large angle relative to the optical axis. By doing so, bright-field illumination with less illumination unevenness can be achieved in the case of an incidence angle smaller than the capturing angle of the objective lens, dark-field illumination with emphasized microstructures can be achieved in the case of an incidence angle larger than the capturing angle of the objective lens, and furthermore, oblique illumination that allows the sample to appear stereoscopic can be achieved in the case of an incidence angle equivalent to the capturing angle of the objective lens.

In the above-described aspect, the light source unit may be capable of simultaneously emitting illumination light from different positions in the circumferential direction of the objective lens.

By doing so, illumination light is simultaneously radiated from a plurality of positions in the circumferential direction of the objective lens, thus making it possible to reduce illumination unevenness.

In the above-described aspect, the light source unit may include a plurality of light sources that are arrayed around the objective lens and that can be lit up independently.

By doing so, the circumferential-direction position of illumination light can be determined by lighting up any of the plurality of light sources. Also, an image of the sample irradiated from different directions can be acquired by switching the circumferential-direction position of the light source to be lit up. In particular, in the case of the above-described oblique illumination, images with different shading patterns can be acquired.

In the above-described aspect, the light source unit may include light sources disposed below the sample and a light-blocking member having an opening that, of the illumination light from the light sources, transmits only illumination light from a particular radial position.

By doing so, the illumination light from the light sources is blocked by the light-blocking member, thus allowing only illumination light passing through the opening to be reflected above the sample and to be incident on the sample. Therefore, the direction or angle of reflection light that is made incident on the sample can be changed by adjusting the position of the opening in the light-blocking member, without switching the lit-up positions of the light sources.

In the above-described aspect, the light source unit may include a diffusing plate for diffusing illumination light.

By doing so, the sample can be irradiated with illumination light that is uniformly diffused by the diffusing plate.

In the above-described aspect, the sample may be accommodated in a container formed of an optically transparent material, and the illumination light may be reflected at an inner surface of a top plate of the container disposed above the sample.

By doing so, merely by disposing, above the light source unit and the image acquisition optical system, the container that accommodates the sample therein and that has a top plate, illumination light emitted from the light source unit can be reflected at the inner surface of the top plate of the container and can be radiated on the sample in the container.

In the above-described aspect, the illumination light may be reflected by a reflecting member disposed above the sample.

By doing so, in a case where a sample accommodated in a container or a cell culture bag without a top plate, like a petri dish (without a lid), is to be observed, by disposing the reflecting member above the sample, illumination light emitted from the light source unit can be reflected at the reflecting member and can be radiated on the sample in the container.

In the above-described aspect, the sample may be immersed in a solution, and the illumination light may be reflected at an upper liquid surface of the solution.

By doing so, in a case where a sample accommodated in a container without a top plate or a container in which no reflecting members can be disposed is to be observed, illumination light emitted from the light source unit can be reflected at the liquid surface of the solution and can be radiated on the sample in the container.

Another aspect of the present invention is an observation method including: an emission step of emitting illumination light from below a sample to thereabove; a reflection step of reflecting, above the sample, the illumination light emitted in the emission step; a transmission step of causing the illumination light reflected in the reflection step to pass through the sample; and an image acquisition step of acquiring, below the sample, an image of transmitted light that has passed through the sample in the transmission step.

### {Advantageous Effects of Invention}

The present invention affords an advantage in that the burden of a confirmation task at the time of cell culturing can be reduced. Furthermore, the culture state of cells in a culture container can be confirmed without opening the door of an incubator and taking out the culture container, thus suppressing an influence of environmental changes on the sample.

### {Brief Description of Drawings}

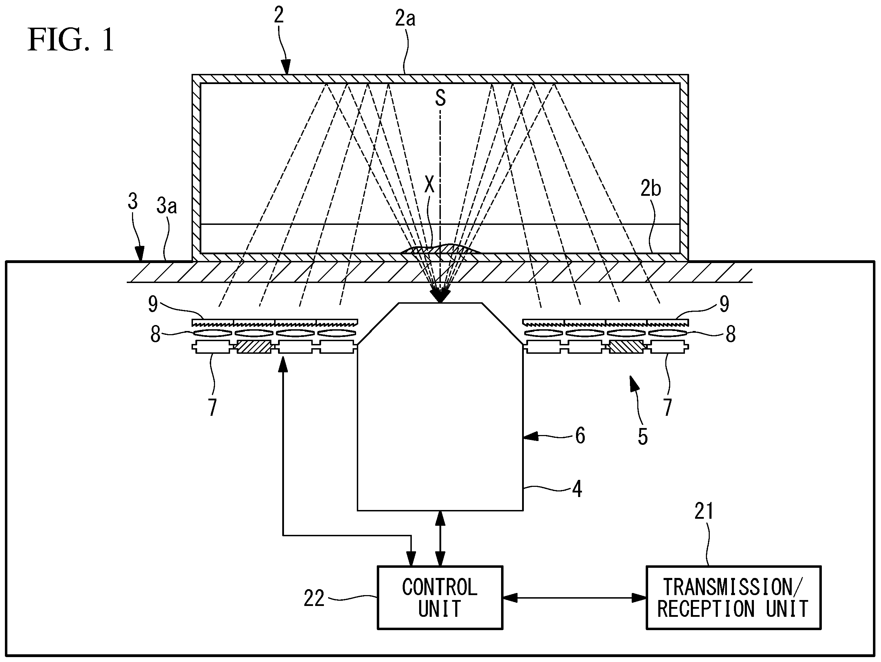

{Fig. 1}
   Fig. 1 is a longitudinal sectional view depicting an observation device of the present invention.
{Fig. 2}
   Fig. 2 is a diagram depicting the observation device of the present invention.
{Fig. 3}
   Fig. 3 is a diagram depicting a modification of the observation device of the present invention.
{Fig. 4A}
   Fig. 4A is a diagram depicting a modification of the observation device of the present invention.
{Fig. 4B}
   Fig. 4B is a diagram depicting a modification of the observation device of the present invention.
{Fig. 4C}
   Fig. 4C is a diagram depicting a modification of the observation device of the present invention.
{Fig. 5A}
   Fig. 5A is a diagram depicting a modification of the observation device of the present invention.
{Fig. 5B}
   Fig. 5B is a diagram depicting a modification of the observation device of the present invention.
{Fig. 6}
   Fig. 6 is a diagram depicting a modification of the observation device of the present invention.
{Fig. 7}
   Fig. 7 is a diagram depicting a modification of the observation device of the present invention.
{Fig. 8}
   Fig. 8 is a diagram depicting a modification of the observation device of the present invention.
{Fig. 9}
   Fig. 9 is a diagram depicting a modification of the observation device of the present invention.
{Fig. 10}
   Fig. 10 is a diagram depicting a culture observation system according to a first embodiment of the present invention.
{Fig. 11}
   Fig. 11 is a diagram depicting a culture observation system according to a second embodiment of the present invention.
{Fig. 12}
   Fig. 12 is a diagram depicting a modification of the observation device of the present invention.
{Fig. 13}
   Fig. 13 is a partial longitudinal sectional view depicting an observation device according to a reference embodiment of the present invention.
{Fig. 14}
   Fig. 14 is a plan view depicting one example of the arrangement of LED light sources in a light source unit of the observation device in Fig. 13.
{Fig. 15}
   Fig. 15 is a modification of the observation device in Fig. 13 in the form of a partial longitudinal sectional view depicting a case where illumination light is restricted by a light-blocking member.
{Fig. 16A}
   Fig. 16A is an example of the light-blocking member in Fig. 15 in the form of a plan view depicting a case where the light-blocking member has a single circular opening.
{Fig. 16B} Fig. 16B is an example of the light-blocking member in Fig. 15 in the form of a plan view depicting a case where the radial position of the opening differs from that in Fig. 16A {Fig. 16C}
Fig. 16C is an example of the light-blocking member in Fig. 15 in the form of a plan view depicting a case where the light-blocking member has two openings.
{Fig. 17A}
   Fig. 17A is another example of the light-blocking member in Fig. 15 in the form of a plan view depicting a case where the light-blocking member has a fan-shaped opening.
{Fig. 17B}
   Fig. 17B is another example of the light-blocking member in Fig. 15 in the form of a plan view depicting a case where the light-blocking member has a ring-shaped opening.
{Fig. 18}
   Fig. 18 is a partial longitudinal sectional view depicting another modification of the observation device in Fig. 13.
{Fig. 19}
   Fig. 19 is a partial longitudinal sectional view depicting another modification of the observation device in Fig. 13.
{Fig. 20}
   Fig. 20 is a partial longitudinal sectional view depicting another modification of the observation device in Fig. 13.
{Fig. 21}
   Fig. 21 is a partial longitudinal sectional view depicting another modification of the observation device in Fig. 13.

### {Description of Embodiments}

### (First embodiment)

A culture observation system 100 according to a first embodiment of the present invention will now be described with reference to the drawings.

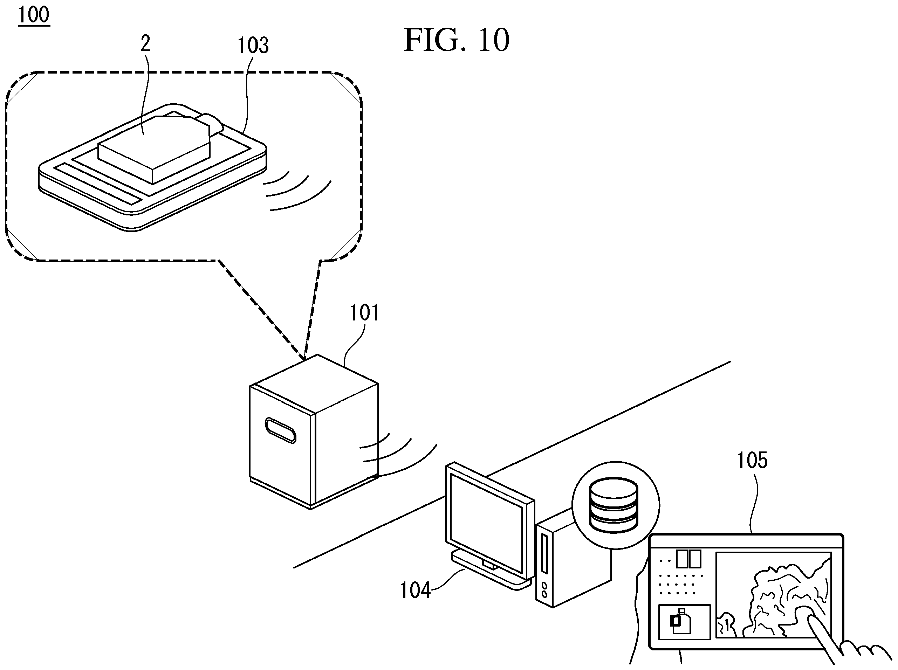

As shown in Fig. 10, the culture observation system 100 according to this embodiment includes: an observation device 103 for monitoring a sample (e.g., cells) in a culture container 2 disposed in an incubator 101; a station server 104 that is disposed outside the incubator 101 and that transmits and receives information to and from the observation device 103 in the incubator 101; and a user terminal 105 for transmitting and receiving information to and from the station server 104.

As shown in Fig. 1, the observation device 103 according to this embodiment includes: a base 3 for mounting the culture container 2 in which a sample X, such as cells, is accommodated together with a culture solution; and a light source unit 5, an image acquisition unit 6, a transmission/reception unit 21, and a control unit 22 that are provided in the base 3.

The culture container 2 is, for example, a flask for cell culturing and is formed of an optically transparent material.

The base 3 is, for example, a housing, and the light source unit 5, the image acquisition unit 6, transmission/reception unit 21, and the control unit 22 are provided in the interior of the housing. At least a portion of the top surface of the base 3 includes a mounting surface 3a formed of an optically transparent material (e.g., glass), and the culture container 2 is mounted on the mounting surface 3a. Because the interior of the incubator becomes very humid, the base 3 preferably has a waterproof structure.

The image acquisition unit 6 includes: an objective lens 4 that is disposed in the interior of the base 3 and below the mounting surface 3a and that collects light passing through the mounting surface 3a from thereabove; and an image acquisition optical system for acquiring an image of the light passing through the sample X.

The light source unit 5 is disposed radially outward of the objective lens 4 and emits illumination light upward so as to pass through the mounting surface 3a.

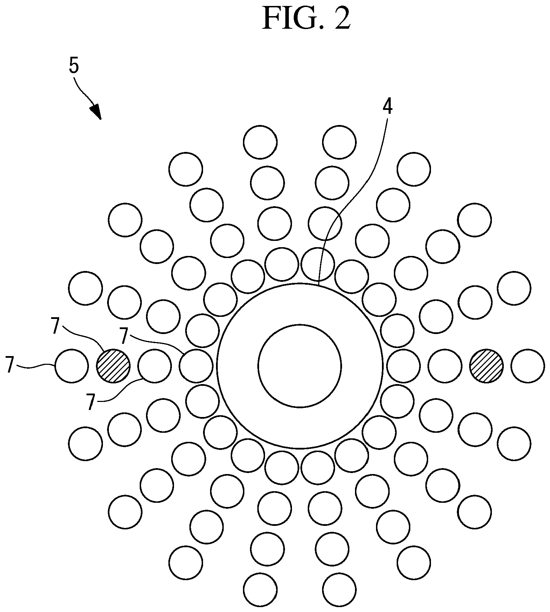

As shown in Figs. 1 and 2, the light source unit 5 includes: a plurality of LED light sources (light sources) 7 that are disposed around the objective lens 4 in a manner spaced apart from one another in the circumferential direction and in the radial direction; a plurality of collimating lenses 8 that are disposed in correspondence with the respective LED light sources 7 and that convert illumination light generated at the respective LED light sources 7 into substantially collimated light; and diffusing plates 9 for diffusing the illumination light collimated by the collimating lenses 8.

The light source unit 5 is configured to be capable of lighting up particular LED light sources 7 independently (in Figs. 1 and 2, lit-up LED light sources 7 are indicated in hatching).

More specifically, by lighting up only LED light sources 7 at different positions in the radial direction of the objective lens, the angles of illumination light that is incident on the objective lens 4 as shown by solid lines in Fig. 1 can be changed as shown by dashed lines, said illumination light having passed upward through the mounting surface 3a and a bottom surface 2b of the container 2, having been reflected at the inner surface of a top plate 2a of the container 2, and having passed obliquely downward through the sample X, the bottom surface 2b of the container 2, and the mounting surface 3a.

In addition, by lighting up only LED light sources 7 at specific positions in the circumferential direction of the objective lens 4, the sample X can be irradiated only in particular directions in the circumferential direction. In addition, as shown in Fig. 2, the sample X can be irradiated with illumination light with reduced illumination unevenness by lighting up the LED light sources 7 disposed in two or more directions in the circumferential direction of the objective lens 4, particularly in directions axially symmetrical with respect to the optical axis of the objective lens 4.

The light source unit 5 may include: a plurality of the LED light sources (light sources) 7 that are disposed around the objective lens 4 in a manner spaced apart from one another only in the circumferential direction; a plurality of collimating lenses 8 that are disposed in correspondence with the respective LED light sources 7 and that convert illumination light generated at the respective LED light sources 7 into substantially collimated light; and diffusing plates 9 for diffusing the illumination light collimated by the collimating lenses 8.

Four LED light sources (light sources) 7, four collimating lenses 8, and four diffusing plates 9 may be provided in a manner spaced apart from one another by 90° in the circumferential direction.

An observation method in which the observation device 103 according to this embodiment with the above-described structure is used will be described below.

In order to observe the transparent sample X, such as cells, by using the observation device 103 according to this embodiment, the sample X is accommodated in the container 2, and the container 2 is mounted on the mounting surface 3a of a stage 3 with the bottom surface 2b face down in a state where the sample X is attached to the bottom surface 2b, as shown in Fig. 1.

Then, arbitrary LED light sources 7 of the light source unit 5 are turned on in this state to generate illumination light. Illumination light generated in the LED light sources 7 is collimated by the collimating lenses 8 disposed in correspondence with the LED light sources 7 and is diffused by the diffusing plates 9, and the illumination light in that state passes upward through the mounting surface and the bottom surface 2b of the container 2 (emission step), is reflected at the inner surface of the top plate 2a of the container 2, and is then radiated obliquely downward onto the sample X (reflection step).

Of the illumination light radiated onto the sample X, transmitted light of the illumination light that has passed through the sample X passes downward through the bottom surface 2b of the container 2 and the mounting surface and is incident on the objective lens 4 (transmission step). In this case, illumination light is refracted and scattered due to the shape and index of refraction of the sample X or is dimmed due to the transmittance of the sample X and is then collected by the objective lens 4 in the form of transmitted light carrying information about the sample X, thus causing an image capturing element (not shown in the figure) to acquire an image of the transmitted light (image acquisition step).

In this manner, the observation device 103 according to this embodiment affords an advantage in that because the light source unit 5 and the image acquisition optical system including the objective lens 4 are disposed below the sample X, the light source unit 5 and the image acquisition optical system 6 can be aggregated on only one side of the sample X, thereby making the device thin. There is another advantage in that, even in the observation device 103 that is made thin in this manner, observation is possible by acquiring an image of transmitted light without labeling a subject, such as cells.

Another advantage is that, as a result of being emitted from radially outward of the objective lens 4 and being reflected at the inner surface of the top plate 2a of the container 2, illumination light from the light source unit 5 is radiated obliquely downward onto the sample X and is collected by the objective lens 4, and therefore, light and dark areas can be formed on an image of the sample X by appropriately setting the angle of incidence on the sample X, thereby making it possible to acquire an easy-to-see image even for a transparent subject, such as cells.

In addition, because the light source unit 5 includes the plurality of LED light sources 7 that are arranged around the objective lens 4 in the radial direction and that can be lit up independently in this embodiment, the irradiation angles of illumination light incident on the sample X can be changed as shown by dashed lines in Fig. 1 by making the radial positions of the lit-up LED light sources 7 different. By doing so, bright-field illumination with less illumination unevenness can be achieved in the case of an incidence angle smaller than the capturing angle of the objective lens 4, dark-field illumination with emphasized microstructures can be achieved in the case of an incidence angle larger than the capturing angle of the objective lens 4, and furthermore, oblique illumination that allows the sample X to appear stereoscopic can be achieved in the case of an incidence angle equivalent to the capturing angle of the objective lens 4.

In addition, because the light source unit 5 is provided with the plurality of LED light sources 7 that are arranged around the objective lens 4 in the circumferential direction thereof and that can be lit up independently in this embodiment, the irradiation directions of illumination light incident on the sample X can be changed by making the circumferential-direction positions of the lit-up LED light sources 7 different. By doing so, the appearance of a formed image of the sample X can be changed by changing the direction of shading of the image.

In addition, as shown in Fig. 2, there is an advantage in that a less uneven image of the sample X can be acquired by reducing illumination unevenness by simultaneously lighting up a plurality of LED light sources 7 at different positions in the circumferential direction, particularly by simultaneously lighting up a plurality of LED light sources 7 that are axially symmetrically disposed.

In addition, because the diffusing plates 9 are provided in correspondence with the respective LED light sources 7 in this embodiment, illumination light emitted from the LED light sources 7 is uniformly diffused, allowing the sample X to be irradiated with illumination light that has less illumination unevenness and that has more uniform intensity.

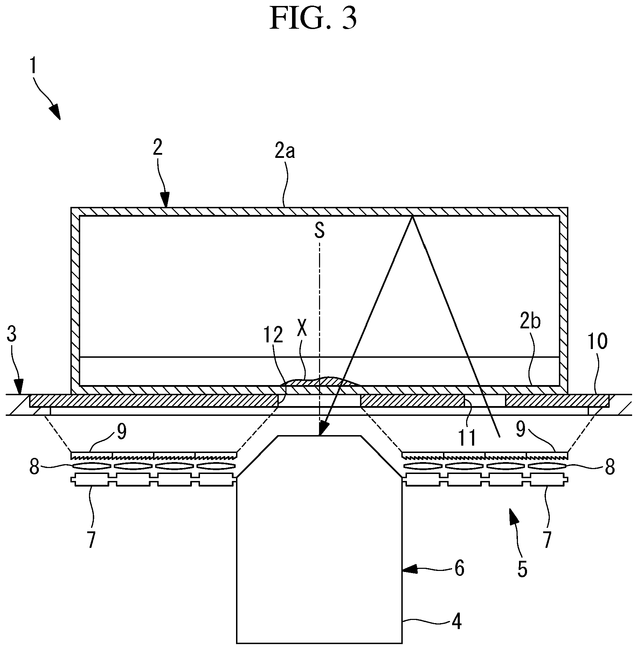

Note that although the irradiation angles, the irradiation directions, etc. of illumination light are switched by arranging the plurality of LED light sources 7 in an array and by lighting up the plurality of LED light sources 7 independently in this embodiment, instead of this, the light source unit 5 may include: the light sources 7 that are disposed around the objective lens 4; and a light-blocking member 10 that is disposed above the light sources 7 and that blocks illumination light from the light sources 7, as shown in Fig. 3, Figs. 4A to 4C, and Figs. 5A and 5B.

More specifically, the light-blocking member 10 includes: an opening 11 formed at a portion in the circumferential direction thereof or at a portion in the radial direction thereof; and a transmission hole 12 for transmitting the light that has been reflected at the inner surface of the top plate 2a of the container 2 and that has passed through the sample X, thereby making it possible to change the irradiation angles and the irradiation directions of illumination light by adjusting the position of the opening 11 by replacing the light-blocking member 10. In this case, although the light source unit 5 may be realized by a light source unit provided with the LED light sources 7, the collimating lenses 8, and the diffusing plates 9 that are arranged in an array as described above, the function of switching the light-emission positions of illumination light is not necessary, and a light source unit provided with any light sources may be employed as long as the light sources can emit illumination light from an area wider than the opening 11.

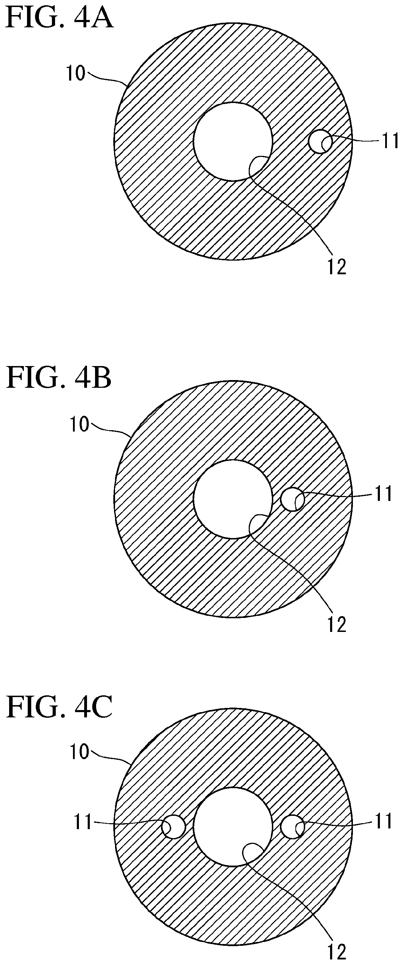

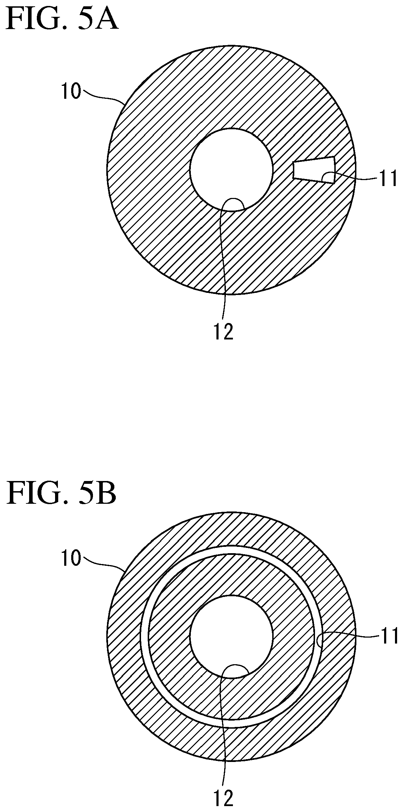

Figs. 4A to 4C show examples where circular openings 11 are provided at different radial directions and where different numbers of openings 11 are provided. Fig. 5A and Fig. 5B show a fan-shaped opening 11 and a ring-shaped opening 11, respectively. For the openings 11, openings having any sizes, positions, and shapes can be employed.

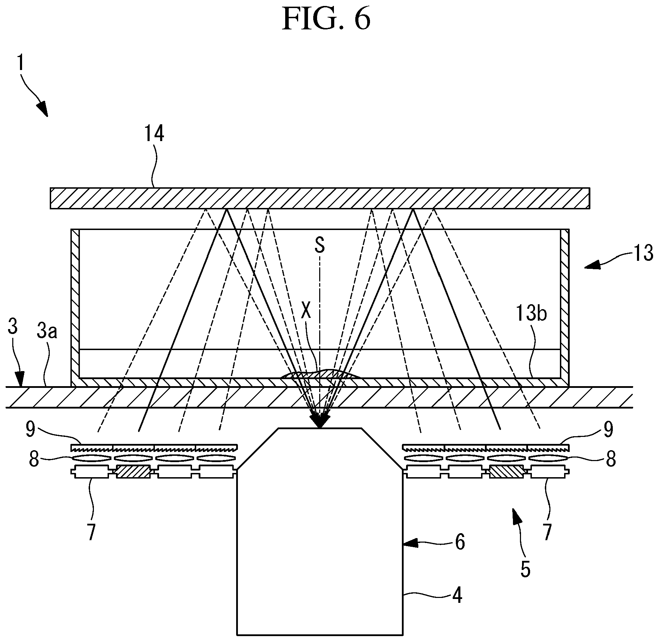

Although the sample X is accommodated in the container 2, such as a cell culture flask, having the top plate 2a so that illumination light is reflected at the inner surface of the top plate 2a of the container 2 in this embodiment, the present invention is not limited to this. For example, if the sample X is accommodated in a container 13 not having the top plate 2a, such as a petri dish (without a lid), serving as the container 2, a reflecting member 14, such as a mirror, may be disposed at a position where the reflecting member 14 covers the top opening of the petri dish, as shown in Fig. 6, so that illumination light that has passed upward through a bottom surface 13b can be reflected by the reflecting member 14. The reflecting member 14 may be provided so as to be capable of being inserted and removed at a position above the sample X by means of a linear movement or swiveling movement.

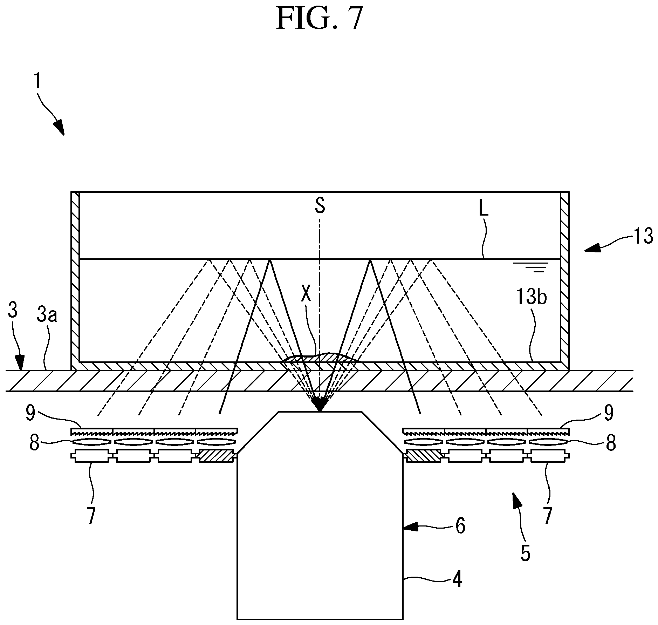

If the sample X is accommodated in the container 13 not having the top plate 2a, such as a petri dish (without a lid), serving as the container 2, a solution (e.g., culture medium, phosphoric acid buffer solution, etc.) L may be poured into the container 13 and the sample X may be immersed into the solution, as shown in Fig. 7, so that illumination light that has passed upward through the bottom surface 13b can be reflected at the upper liquid surface of the solution. Also in the case where the sample X is accommodated in the container 2 having the top plate 2a, the solution (e.g., culture medium, phosphoric acid buffer solution, etc.) L may be poured into the container 2, and the sample X may be immersed into the solution.

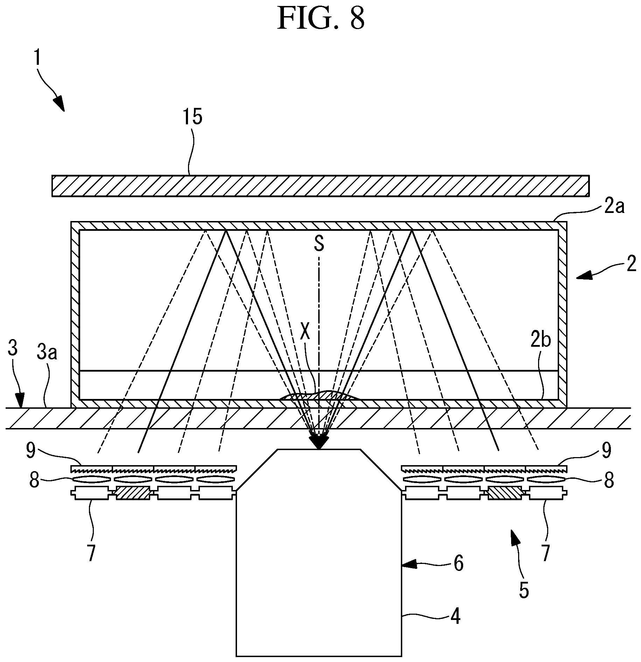

In addition, in this embodiment, a light-blocking member 15 formed of a material for blocking light may be provided above the top plate 2a, as shown in Fig. 8.

By doing so, because ambient light from outside is blocked by the light-blocking member 15, entry of ambient light into the container 2 via the top plate 2a is suppressed, thereby allowing efficient observation.

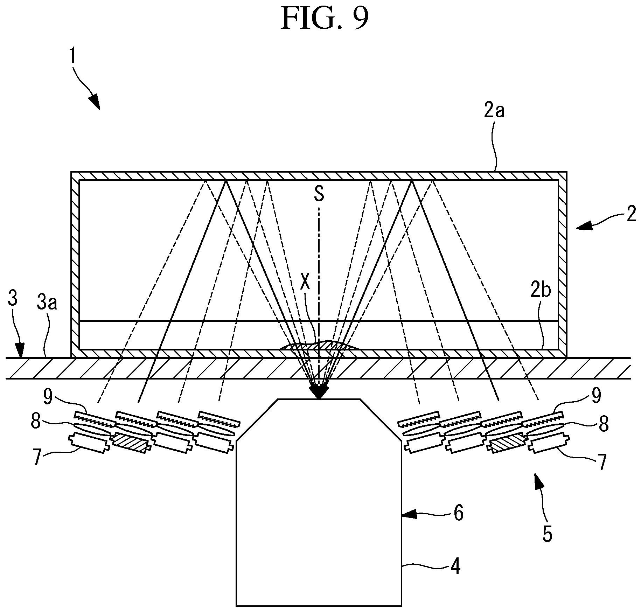

In addition, although this embodiment has been described by way of an example of the light source unit 5 in which the LED light sources 7, the collimating lenses 8, and the diffusing plates 9 are disposed substantially horizontally so as to extend along a glass plate 3a, instead of this, the LED light sources 7, the collimating lenses 8, and the diffusing plates 9 may be disposed obliquely relative to an optical axis S, as shown in Fig. 9.

By doing so, it is possible to suppress a loss in illumination light emitted from the LED light sources 7 and efficiently irradiate the sample X with illumination light.

In addition, although this embodiment has been described by way of an example of the light source unit 5 provided with the diffusing plates 9, the light source unit 5 need not be provided with the diffusing plates 9.

The transmission/reception unit 21 is configured to transmit and receive, via wires or wirelessly, information to and from the station server 104 placed outside the incubator. Via wires or wirelessly, the transmission/reception unit 21 transmits an image acquired by the image acquisition unit 6 to the external station server 104, receives information from the station server 104, and transmits this information to the control unit 22.

The control unit 22 operates a light source unit 3, the image acquisition unit 6, and the transmission/reception unit 21 on the basis of information from the station server 104.

Alternatively, the control unit 22 may be provided with, for example, a timer (not shown in the figure) so as to operate the light source unit 3, the image acquisition unit 6, and the transmission/reception unit 21 periodically.

The station server 104 is disposed outside the incubator and transmits and receives information to and from the observation device 103 in the incubator via wires or wirelessly. The station server 104 also transmits and receives information to and from the user terminal 105 via wires or wirelessly.

The station server 104 receives sample data (e.g., images) transmitted from the observation device and transmits this sample data to the user terminal 105. In addition, the station server 104 transmits information to the observation device 103 in the incubator on the basis of information transmitted from the user terminal 105.

The station server 104 may include display means (monitor) and may display, on this display means, sample data transmitted from the observation device. In this case, the user terminal 105 need not be provided.

The station server 104 may include an input unit (e.g., keyboard, mouse) (not shown in the figure) and may transmit information input with this input unit to the observation device in the incubator. In this case, the user terminal 105 need not be provided.

The station server 104 is, for example, a PC.

The user terminal 105 includes a display unit and an input unit and transmits and receives information to and from the station server 104 wirelessly.

The user terminal 105 receives sample data transmitted from the station server 104 and displays this sample data on the display unit of the user terminal 105. The user terminal 105 also transmits, to the station server 104, information input to the input unit of the user terminal 105.

The user terminal 105 is, for example, a PC, a smartphone, or a tablet.

### (Second embodiment)

A culture observation system 200 according to a second embodiment of the present invention will be described below with reference to the drawings.

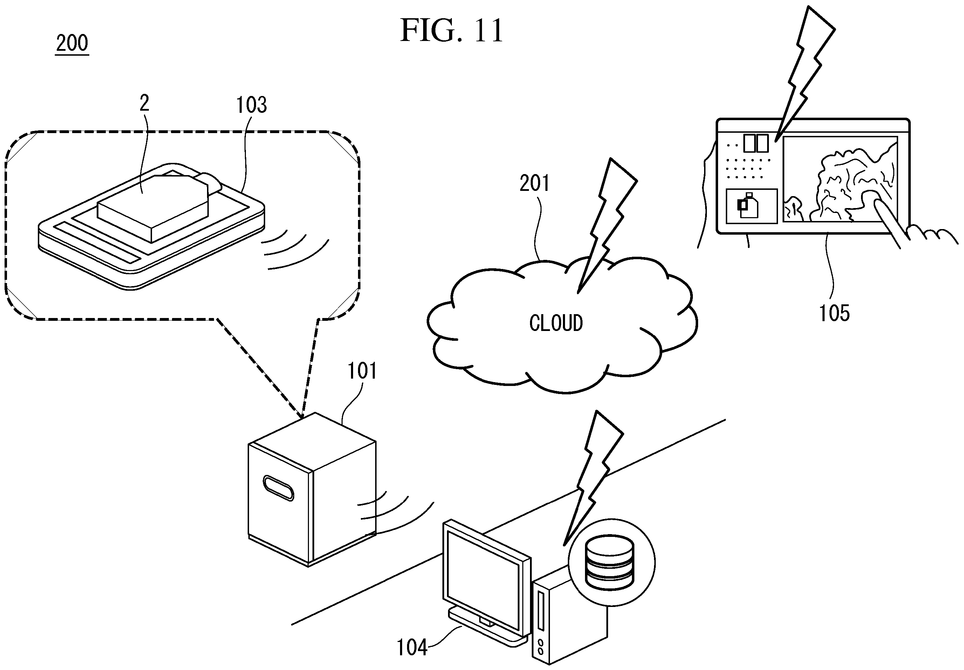

As shown in Fig. 11, the culture observation system 200 according to this embodiment includes: an observation device 103 for monitoring a sample (e.g., cells) in a culture container 2 disposed in an incubator 101; a station server 104 that is disposed outside the incubator 101 and that transmits and receives information to and from the observation device 103 in the incubator 101; a cloud server 201 for transmitting and receiving information to and from this station server 104 via the Internet; and a user terminal 105 for transmitting and receiving information to and from this cloud server 201 via the Internet.

The second embodiment differs from the first embodiment in that the station server 104 and the user terminal 105 transmit and receive information via the cloud server 201.

The observation device 103 is the same as that in the first embodiment.

The station server 104 is disposed outside the incubator 101 and transmits and receives information to and from the observation device 103 in the incubator 101 via wires or wirelessly. In addition, the station server 104 transmits and receives information to and from the user terminal 105 via the cloud server 201 on the Internet.

The station server 104 receives image data transmitted from the observation device 103 and transmits this image data to the user terminal 105. The station server 104 also transmits information to the observation device 103 in the incubator 101 on the basis of information transmitted from the user terminal 105.

Transmission and reception of information from the station server 104 to the cloud server 201 may be performed at each instruction from the user terminal 105 or may be automatically performed at predetermined intervals and/or for predetermined image data.

The station server 104 is, for example, a PC.

The user terminal 105 includes a display unit and an input unit and transmits and receives information to and from the cloud server 201 on the Internet and to and from the station server 104 via the cloud server 201 on the Internet.

The user terminal 105 receives sample data (e.g., images) transmitted from the station server 104 or the cloud server 201 and displays this sample data on the display unit of the user terminal 105. In addition, the user terminal 105 transmits, to the station server 104, information input to the input unit of the user terminal 105.

The user terminal 105 is, for example, a PC, a smartphone, or a tablet.

Sample data uploaded from the station server 104 to the cloud server 201 may include not only images but also: image analysis data (analysis results, analysis conditions, etc.); project data of the observation device 103 (operator name, male or female, age, image acquisition conditions, etc.); operation information log data of the observation device 103 (device suspension, restart, task processing information, accumulated operating time, etc.); task information log data (culture medium replacement, subculture operation, etc.); culture information data (container type, cell type, culture medium name, blood serum name, etc.); culture environmental data (culture medium color, component information, temperature, humidity, etc.); etc.

By displaying these data appropriately in association with image data, it is possible to perceive the cell state in the culture from more diversified perspectives and more quantitatively, compared with conventional cases where a worker perceives the cell state by taking the cell container from the incubator and observing the cell condition with a microscope or the like.

In addition, sample data uploaded from the station server 104 may be analyzed and visualized on the cloud server 201. By doing so, without changing the configurations and the specifications of the observation device 103 and the station server 104, it is possible to perform analysis and visualization that are impossible with the observation device 103 and the station server 104.

In each of the above-described embodiments, the observation device may include moving means for moving the light source unit and the image acquisition unit.

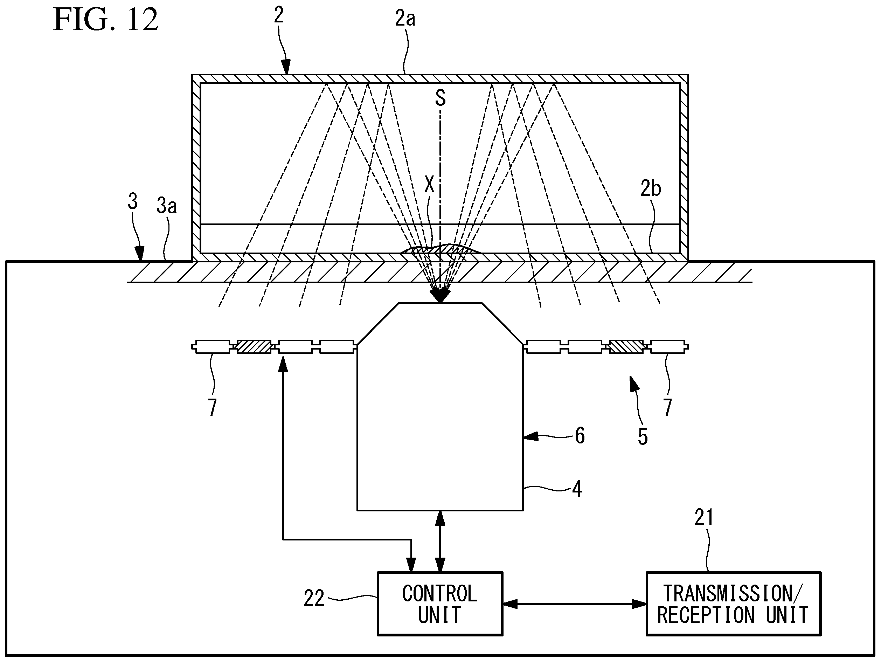

Although each of the above-described embodiments has been described by way of an example where the observation device employs the light source unit 5 including the collimating lenses 8 and the diffusing plates 9, the light source unit 5 need not include the collimating lenses 8 and the diffusing plates 9 as shown in, for example, Fig. 12.

Although the observation device in each of the above-described embodiments includes the light source unit disposed in the base, the light source unit may be disposed above the container or on a side surface of the container.

### (Reference embodiment)

An observation device 100 according to one embodiment of the present invention will be described below with reference to the drawings.

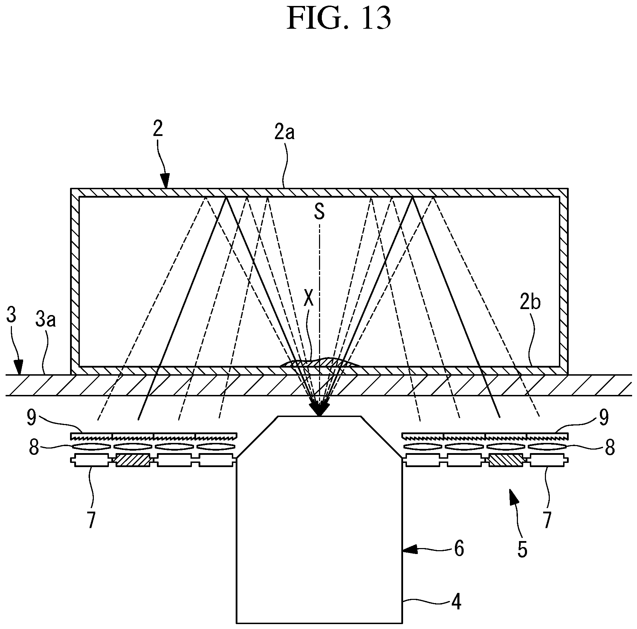

As shown in Fig. 13, the observation device 100 according to this embodiment: includes a stage 3 for mounting a container 2 in which a sample X is accommodated and an objective lens 4 that is disposed below this stage 3 and that collects light passing through the stage 3 from thereabove; and also includes an image acquisition optical system 6 for acquiring an image of light passing through the sample X and a light source unit 5 that is disposed radially outward of the objective lens 4 and that emits illumination light upward so as to pass through the stage 3.

An optically transparent material, such as a glass plate 3a, is disposed on the stage 3 so as to cover the space above the objective lens 4 and the light source unit 5, and the container 2 is mounted on the top surface of the glass plate 3a.

The container 2 is, for example, a cell culture flask having a top plate 2a and is formed of an optically transparent resin as a whole.

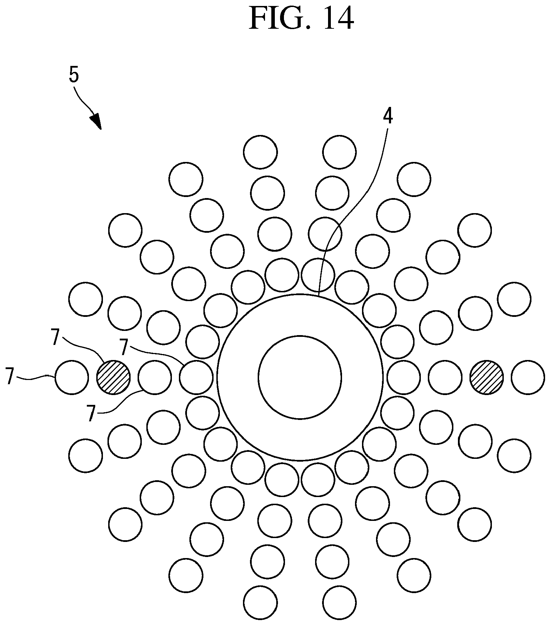

As shown in Figs. 13 and 14, the light source unit 5 includes: a plurality of LED light sources (light sources) 7 that are disposed around the objective lens 4 in a manner spaced apart from one another in the circumferential direction and in the radial direction; a plurality of collimating lenses 8 that are disposed in correspondence with the respective LED light sources 7 and that convert illumination light generated at the respective LED light sources 7 into substantially collimated light; and diffusing plates 9 for diffusing the illumination light collimated by the collimating lenses 8.

The light source unit 5 is configured to be capable of lighting up particular LED light sources 7 independently (in Figs. 13 and 14, lit-up LED light sources 7 are indicated in hatching).

More specifically, by lighting up only LED light sources 7 at different positions in the radial direction of the objective lens 4, the angles of illumination light that is incident on the objective lens 4 as shown by solid lines in Fig. 13 can be changed as shown by dashed lines, said illumination light having passed upward through the glass plate 3a and a bottom surface 2b of the container 2, having been reflected at the inner surface of the top plate 2a of the container 2, and having passed obliquely downward through the sample X, the bottom surface 2b of the container 2, and the glass plate 3a.

In addition, by lighting up only LED light sources 7 at specific positions in the circumferential direction of the objective lens 4, the sample X can be irradiated only in particular directions in the circumferential direction. In addition, as shown in Fig. 14, the sample X can be irradiated with illumination light with reduced illumination unevenness by lighting up the LED light sources 7 disposed in two or more directions in the circumferential direction of the objective lens 4, particularly in directions axially symmetrical with respect to an optical axis S of the objective lens 4.

An observation method in which an observation device 1 according to this embodiment with the above-described structure is used will be described below.

In order to observe the transparent sample X, such as cells, by using the observation device 1 according to this embodiment, the sample X is accommodated in the container 2, and the container 2 is mounted on the glass plate 3a of the stage 3 with the bottom surface 2b face down in a state where the sample X is attached to the bottom surface 2b, as shown in Fig. 13.

Then, arbitrary LED light sources 7 of the light source unit 5 are turned on in this state to generate illumination light. Illumination light generated in the LED light sources 7 is collimated by the collimating lenses 8 disposed in correspondence with the LED light sources 7 and is diffused by the diffusing plates 9, and the illumination light in that state passes upward through the glass plate 3a and the bottom surface 2b of the container 2 (emission step), is reflected at the inner surface of the top plate 2a of the container 2, and is then radiated obliquely downward onto the sample X (reflection step).

Of the illumination light radiated onto the sample X, transmitted light of the illumination light that has passed through the sample X passes downward through the bottom surface 2b of the container 2 and the glass plate 3a and is incident on the objective lens 4 (transmission step). In this case, illumination light is refracted and scattered due to the shape and the index of refraction of the sample X or is dimmed due to the transmittance of the sample X and is then collected by the objective lens 4 in the form of transmitted light carrying information about the sample X, thus causing an image capturing element (not shown in the figure) to acquire an image of the transmitted light (image acquisition step).

In this manner, compared with conventional transmitted-light observation devices in which a light source unit and an image acquisition optical system are disposed on both sides with a sample interposed therebetween, the observation device 100 according to this embodiment affords an advantage in that because the light source unit 5 and the image acquisition optical system 6 including the objective lens 4 are disposed below the sample X, the light source unit 5 and the image acquisition optical system 6 can be aggregated on only one side of the sample X, thereby making the device thin. There is another advantage in that, even in the observation device 100 that is made thin in this manner, observation is possible by acquiring an image of transmitted light without labeling a subject, such as cells.

Another advantage is that, as a result of being emitted from radially outward of the objective lens 4 and being reflected at the inner surface of the top plate 2a of the container 2, illumination light from the light source unit 5 is radiated obliquely downward onto the sample X and is collected by the objective lens 4, and therefore, light and dark areas can be formed on an image of the sample X by appropriately setting the angle of incidence on the sample X, thereby making it possible to acquire an easy-to-see image even for a transparent subject, such as cells.

In addition, because the light source unit 5 includes the plurality of LED light sources 7 that are arranged around the objective lens 4 in the radial direction and that can be lit up independently in this embodiment, the irradiation angles of illumination light incident on the sample X can be changed as shown by dashed lines in Fig. 13 by making the radial positions of the lit-up LED light sources 7 different. By doing so, bright-field illumination with less illumination unevenness can be achieved in the case of an incidence angle smaller than the capturing angle of the objective lens 4, dark-field illumination with emphasized microstructures can be achieved in the case of an incidence angle larger than the capturing angle of the objective lens 4, and furthermore, oblique illumination that allows the sample X to appear stereoscopic can be achieved in the case of an incidence angle equivalent to the capturing angle of the objective lens 4.

In addition, because the light source unit 5 is provided with the plurality of LED light sources 7 that are arranged around the objective lens 4 in the circumferential direction thereof and that can be lit up independently in this embodiment, the irradiation directions of illumination light incident on the sample X can be changed by making the circumferential-direction positions of the lit-up LED light sources 7 different. By doing so, the appearance of a formed image of the sample X can be changed by changing the direction of shading of the image.

In addition, as shown in Fig. 14, there is an advantage in that a less uneven image of the sample X can be acquired by reducing illumination unevenness by simultaneously lighting up a plurality of the LED light sources 7 at different positions in the circumferential direction, particularly by simultaneously lighting up a plurality of the LED light sources 7 that are axially symmetrically disposed.

In addition, because the diffusing plates 9 are provided in correspondence with the respective LED light sources 7 in this embodiment, illumination light emitted from the LED light sources 7 is uniformly diffused, allowing the sample X to be irradiated with illumination light that has less illumination unevenness and that has more uniform intensity.

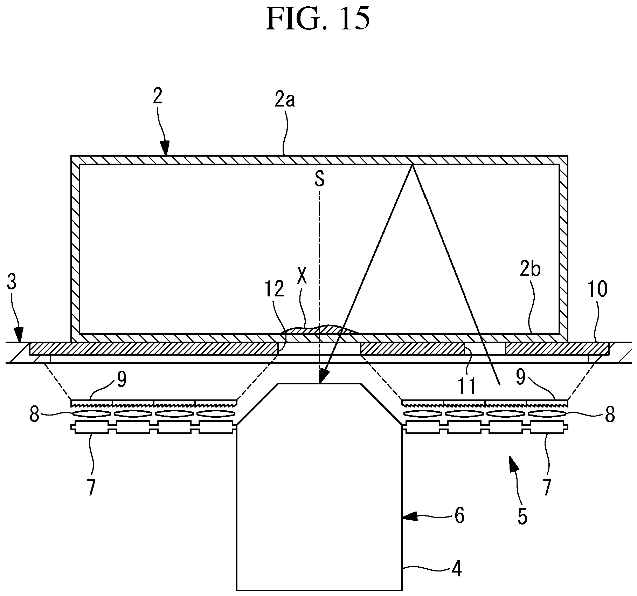

Note that although the irradiation angles, the irradiation directions, etc. of illumination light are switched by arranging the plurality of LED light sources 7 in an array and by lighting up the plurality of LED light sources 7 independently in this embodiment, instead of this, the light source unit 5 may include: light sources 7 that are disposed around the objective lens 4; and a light-blocking member 10 that is disposed above the light sources 7 and that blocks illumination light from the light sources 7, as shown in Fig. 15, Figs. 16A to 16C, and Figs. 17A and 17B.

More specifically, the light-blocking member 10 includes: an opening 11 formed at a portion in the circumferential direction thereof or at a portion in the radial direction thereof; and a transmission hole 12 for transmitting the light that has been reflected at the inner surface of the top plate 2a of the container 2 and that has passed through the sample X, thereby making it possible to change the irradiation angles and the irradiation directions of illumination light by adjusting the position of the opening 11 by replacing the light-blocking member 10. In this case, although the light source unit 5 may be realized by a light source unit provided with the LED light sources 7, the collimating lenses 8, and the diffusing plates 9 that are arranged in an array as described above, the function of switching the light-emission positions of illumination light is not necessary and a light source unit provided with any light sources may be employed as long as the light sources can emit illumination light from an area wider than the opening 11.

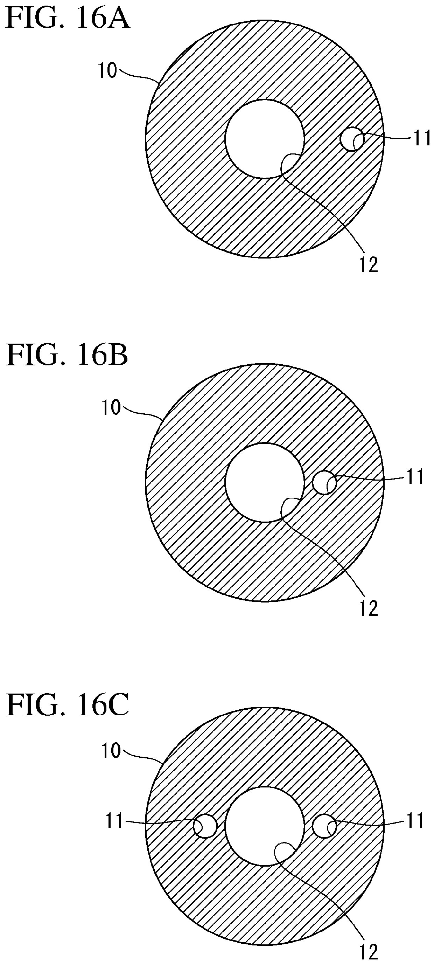

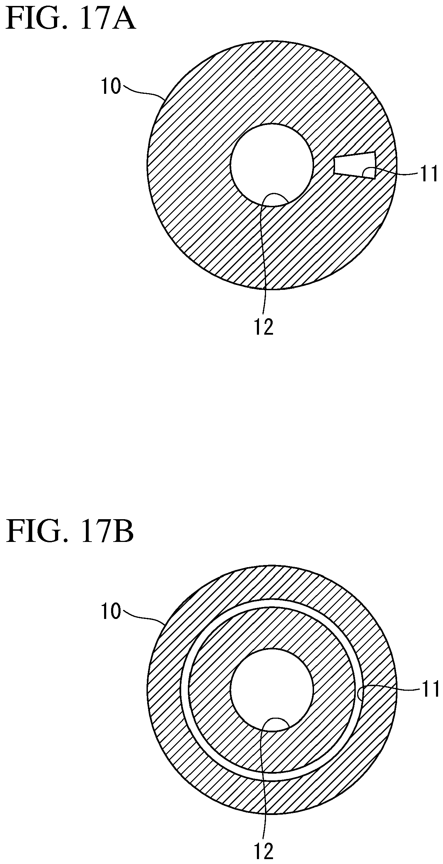

Figs. 16A to 16C show examples where circular openings 11 are provided with different radial directions and different numbers of the openings 11. Fig. 17A and Fig. 17B show a fan-shaped opening 11 and a ring-shaped opening 11, respectively. For the openings 11, openings having any sizes, positions, and shapes can be employed.

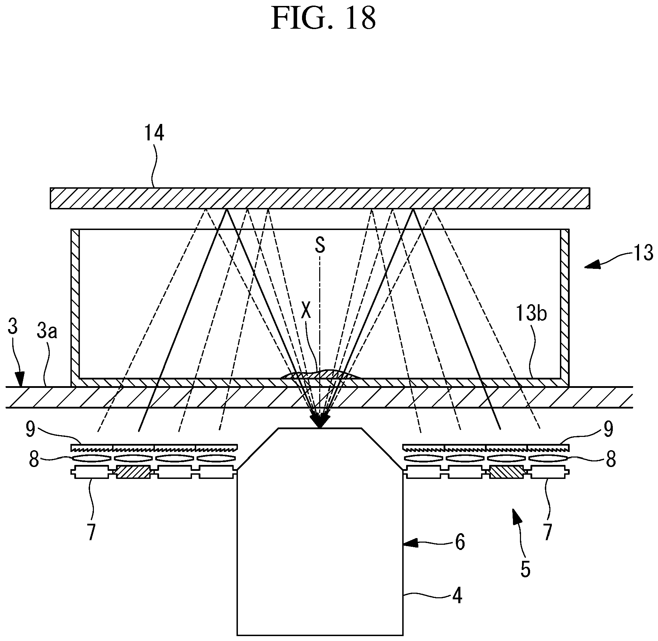

Although the sample X is accommodated in the container 2, such as a cell culture flask, having the top plate 2a so that illumination light is reflected at the inner surface of the top plate 2a of the container 2 in this embodiment, the present invention is not limited to this. For example, if the sample X is accommodated in a container 13 not having the top plate 2a, such as a petri dish (without a lid), serving as the container 2, a reflecting member 14, such as a mirror, may be disposed at a position where the reflecting member 14 covers the top opening of the petri dish, as shown in Fig. 18, so that illumination light that has passed upward through a bottom surface 13b can be reflected by the reflecting member 14. The reflecting member 14 may be provided so as to be capable of being inserted and removed at a position above the sample X by means of a linear movement or swiveling movement.

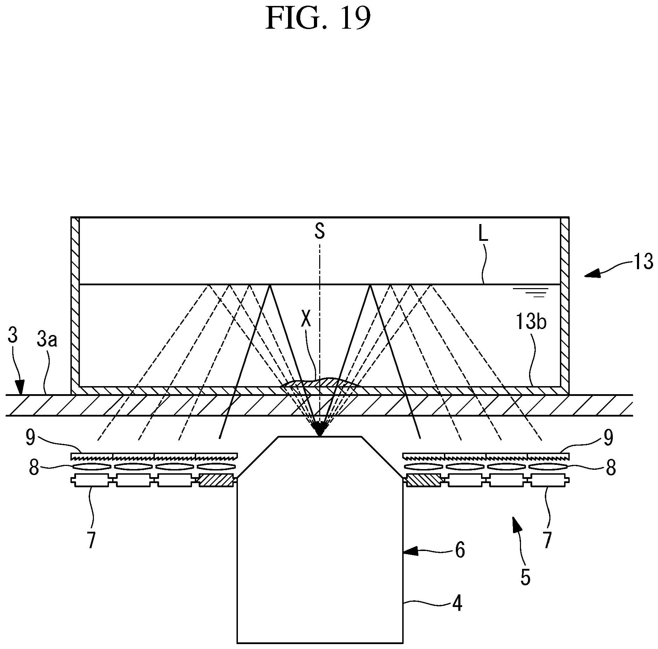

If the sample X is accommodated in the container 13 not having the top plate 2a, such as a petri dish (without a lid), serving as the container 2, a solution (e.g., culture medium, phosphoric acid buffer solution, etc.) L may be poured in the container 13 and the sample X may be immersed into the solution, as shown in Fig. 19, so that illumination light that has passed upward through the bottom surface 13b can be reflected at the upper liquid surface of the solution. Also in the case where the sample X is accommodated in the container 2 having the top plate 2a, the solution (e.g., culture medium, phosphoric acid buffer solution, etc.) L may be poured into the container 2, and the sample X may be immersed in the solution.

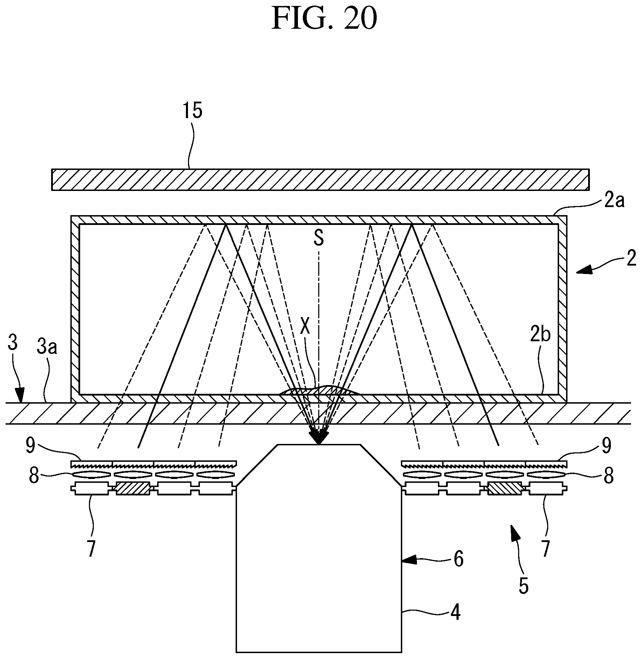

In addition, in this embodiment, a light-blocking member 15 formed of a material for blocking light may be provided above the top plate 2a, as shown in Fig. 20.

By doing so, because ambient light from outside is blocked by the light-blocking member 15, entry of ambient light into the container 2 via the top plate 2a is suppressed, thereby allowing efficient observation.

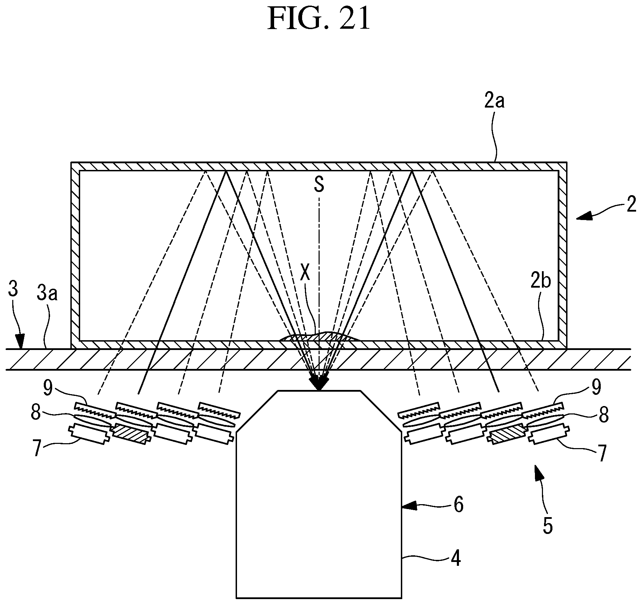

In addition, although this embodiment has been described by way of an example of the light source unit 5 in which the LED light sources 7, the collimating lenses 8, and the diffusing plates 9 are disposed substantially horizontally so as to extend along the glass plate 3a, instead of this, the LED light sources 7, the collimating lenses 8, and the diffusing plates 9 may be disposed obliquely relative to the optical axis S, as shown in Fig. 21.

By doing so, it is possible to suppress loss in illumination light emitted from the LED light sources 7 and efficiently irradiate the sample X with illumination light.

In addition, although this embodiment has been described by way of an example of the light source unit 5 provided with the collimating lenses 8 and the diffusing plates 9, the light source unit 5 need not be provided with the collimating lenses 8 and the diffusing plates 9.

According to this embodiment, the following observation device can be provided.

### (Item 1)

An observation device includes: a light source unit for emitting illumination light from below a sample to thereabove; and an image acquisition optical system for acquiring, below the sample, an image of transmitted light that has passed through the sample as a result of the illumination light emitted from the light source unit being reflected above the sample.

### (Item 2)

The observation device may be an observation device wherein the image acquisition optical system includes an objective lens for collecting transmitted light that has passed through the sample, and the light source unit emits illumination light from radially outward of the objective lens to above the sample.

### (Item 3)

The observation device may be an observation device wherein the light source unit can independently emit illumination light from different positions in the radial direction of the objective lens.

### (Item 4)

The observation device may be an observation device wherein the light source unit can simultaneously emit illumination light from different positions in the circumferential direction of the objective lens.

### (Item 5)

The observation device may be an observation device wherein the light source unit includes a plurality of light sources that are arrayed around the objective lens and that can be lit up independently.

### (Item 6)

The observation device may be an observation device wherein the light source unit includes light sources disposed below the sample and a light-blocking member having an opening that, of the illumination light from the light sources, transmits only illumination light from a particular radial position.

### (Item 7)

The observation device may be an observation device wherein the light source unit includes a diffusing plate for diffusing illumination light.

### (Item 8)

The observation device may be an observation device wherein the sample is accommodated in a container formed of an optically transparent material, and the illumination light is reflected at an inner surface of a top plate of the container disposed above the sample.

### (Item 9)

The observation device may be an observation device wherein the illumination light is reflected by a reflecting member disposed above the sample.

### (Item 10)

The observation device may be an observation device wherein the sample is immersed in a solution, and the illumination light is reflected at an upper liquid surface of the solution.

According to this embodiment, the following observation method is provided.

An observation method includes: an emission step of emitting illumination light from below a sample to thereabove; a reflection step of reflecting, above the sample, the illumination light emitted in the emission step; a transmission step of causing the illumination light reflected in the reflection step to pass through the sample; and an image acquisition step of acquiring, below the sample, an image of transmitted light that has passed through the sample in the transmission step.

### {Reference Signs List}

- 1: Observation device
- 2: Container
- 2a: Top plate
- 4: Objective lens
- 5: Light source unit
- 6: Image acquisition optical system
- 7: LED light source
- 9: Diffusing plate
- 10: Light-blocking member
- 11: Opening
- 14: Reflecting member
- 21: Transmission/reception unit
- 22: Control unit
- 101: Incubator
- 103: Observation device
- 104: Station server
- 105: User terminal
- 201: Cloud server
- X: Sample

## Claims

1. A culture observation system comprising:
an observation device that is disposed in an incubator and that is used to observe a sample in a culture container;
a station server that is disposed outside the incubator and that transmits and receives information to and from the observation device; and
a terminal for transmitting and receiving information to and from the station server,
wherein the observation device includes:
a light source unit for emitting illumination light from below the sample to thereabove, said sample being accommodated in the container formed of an optically transparent material; and
an image acquisition unit for acquiring, below the sample, an image of transmitted light that has passed through the sample as a result of the illumination light emitted from the light source unit being reflected at an inner surface of a top plate of the container disposed above the sample.

2. The culture observation system according to claim 1, wherein the station server and the terminal transmit and receive information via a cloud server.

3. The culture observation system according to claim 2, wherein transmission and reception of information from the station server to the cloud server are performed according to each instruction from the terminal.

4. The culture observation system according to claim 2, wherein transmission and reception of information from the station server to the cloud server are automatically performed at predetermined intervals and/or for predetermined image data.

5. The observation device according to one of claims 1 to 4,
wherein the image acquisition unit includes an objective lens for collecting the transmitted light that has passed through the sample, and
wherein the light source unit emits illumination light from radially outward of the objective lens to above the sample.

6. The observation device according to claim 5, wherein the light source unit is capable of independently emitting illumination light from different positions in the radial direction of the objective lens.

7. The observation device according to claim 5, wherein the light source unit is capable of simultaneously emitting illumination light from different positions in the circumferential direction of the objective lens.

8. The observation device according to claim 6 or 7, wherein the light source unit includes a plurality of light sources that are arrayed around the objective lens and that can be lit up independently.

9. The observation device according to claim 5, wherein the light source unit includes light sources disposed below the sample and a light-blocking member having an opening that, of the illumination light from the light sources, transmits only illumination light from a particular radial position.

10. The observation device according to one of claims 1 to 9, wherein the light source unit includes a diffusing plate for diffusing illumination light.

11. The observation device according to one of claims 1 to 10, wherein the illumination light is reflected by a reflecting member disposed above the sample.

12. The observation device according to one of claims 1 to 10,
wherein the sample is immersed in a solution, and
the illumination light is reflected at an upper liquid surface of the solution.
